# EUROPEAN PATENT SPECIFICATION

(11) **EP 2 594 294 B1**
(45) Date of publication and mention of the grant of the patent: **14.11.2018**
(21) Application number: 12193062.2
(22) Date of filing: 16.11.2012
(51) Int. Cl.: A61L 2/26, A61C 19/02, A61C 19/00, A61B 90/70, A61B 50/20

(54) **Container for dental and medical instruments**
Behälter für dentale und medizinische Instrumente
Récipient pour instruments dentaires et médicaux

(30) Priority: 18.11.2011 EP 11189746
(43) Date of publication of application: 22.05.2013
(73) Proprietor: Steins, M. H. M. J., 6133 VP Sittard (NL)
(72) Inventor: Steins, M. H. M. J., 6133 VP Sittard (NL)
(74) Representative: Ishiguro, Masaoki

(56) References cited:
- EP-A1- 1 839 683
- WO-A1-2010/128408
- US-A- 4 577 755
- US-A- 5 571 488

## Description

The present invention relates to a container for dental instruments and medical instruments, to be used in a washing apparatus.

Dental instruments and medical instruments must be washed after use before they can be reused. Typically, the used instruments are placed in a container such as a mesh basket. One or more of these containers are placed in a rack, which rack is in turn placed in a washing apparatus. The washing apparatus is set to wash the used instruments by spraying cleaning liquids onto the used instruments. Typically, spray nozzles are provided below the rack, which spray the cleaning liquids upward. An example of such medical instrument washer is described in US6758227.

WO97/17665 discloses an integrated system for reprocessing, i.e. cleaning, contaminated medical instruments. The system comprises at least two reprocessing units adapted for receiving therein a medical instrument carrier, electronic controller means coupled to each of said reprocessing units to control the operation thereof; and a control program for said controller means. WO9717665 discloses an instrument carrier which comprises a mesh-like bottom which is arranged to hold surgical instruments during reprocessing.

WO2008/121792 discloses an apparatus for cleaning and sterilizing a medical instrument. The apparatus comprises a container adapted to receive the medical instrument and a chamber which receives the container. WO2008/121792 discloses an example in which the container comprises an inner basket, having upwardly extending silicone fingers to limit shifting of items within the basket. The basket rests on a bottom, which can either be removable or attached to the basket.

US6534000 discloses a sterilization cassette for dental instruments. The cassette comprises a generally rectangular case having sides with perforations for passage of sterilization gasses. The cassette comprises a plier-mounting bar for mounting dental pliers. The cassette is subjected to ultrasonic bath and heat applications.

These known containers or baskets for medical or dental instruments are for situations in which the containers are immersed in cleaning liquid, such as the sterilization process. A container which gives better cleaning results in the washing process which does not involve immersion of the containers in cleaning liquid is desired.

It is an object of the present invention to provide a container for dental instruments and medical instruments which allows better cleaning with a washing apparatus which is arranged to upwardly spray cleaning liquids.

Accordingly, the present invention provides a container for dental instruments and medical instruments, said instruments having two branches that are pivotably arranged with respect to each other to allow said instruments to assume an open state and a closed state, wherein the container comprises a bottom, two side walls facing each other, a back wall and a front wall connected to the two side walls and facing each other, and an instrument mounting member extending between the two side walls and attached to the side walls, wherein the instrument mounting member comprises a first portion being formed of two bars positioned at the same height with respect to the bottom with a space therebetween, the two bars being configured to enable mounting of said instruments in an open state on the first portion of the instrument mounting member, wherein the back wall and the front wall are arranged at a distance with respect to each other that is in accordance with a space between the two branches of said instruments when in an open state and configured to limit rotational movement of said instruments when mounted in an open state on the first portion of the instrument mounting member, the bottom of the container being provided with an opening and the first portion of the instrument mounting member being arranged above the opening, wherein the opening is configured to allow, in use of the container, cleaning liquid to be sprayed unimpeded and directly onto the instruments that are mounted in an open state on the first portion of the instrument mounting member.

The container according to the present invention is advantageous for cleaning medical or dental instruments which have two branches pivotable with respect to each other, i.e. instruments which may be opened and closed. Examples of such instruments are dental and medical pliers and scissors. Pliers include a number of plier-like instruments, and specifically include hard wire cutters, bird's beak pliers, three-prong pliers, ligature cutters, adjustment pliers, Weingart pliers, hemostats, and other plier-like dental hand tools. Other examples include artery forceps, needle holder, or vascular clamp.

These instruments may advantageously be mounted in an open state on the first portion of the instrument mounting member. The opening at the bottom allows cleaning liquid to be directly sprayed onto the instruments. Unlike the prior art containers which have a (perforated) bottom such as a mesh basket, there is nothing that stops the cleaning liquid from being directly sprayed onto the instruments mounted on the first portion of the instrument mounting member. In comparison, a perforated bottom in prior art containers allows spraying the cleaning liquid to the instruments only via perforation, which reduces the efficiency of the use of the cleaning liquid. Further, according to the container of the present invention, the instruments are maintained in an open state during the washing. Since the instruments are in an open state, it is ensured that the pivot points which tend to accumulate contamination are directly washed by the jet of cleaning liquids from below. This solves the problem of the prior art baskets that the instruments laid on the bottom of a prior art basket may close during the washing, resulting in an insufficient cleaning of the pivot points.

The back wall and the front wall limit the rotational movement of the instruments mounted on the first portion of the instrument mounting member. This prevents the instruments from falling off from the instrument mounting member during the wash. Appropriate distance between the back wall and the front wall for this is about the distance between the handles of relevant instruments in an open state. The suitable distance between the back wall and the front wall depends on the type of the instruments, but is for example 7-12 cm or 8-10 cm.The first portion is preferably positioned substantially at the same distance from the front wall and the back wall, i.e. the distance between the front wall and the bar of the first portion closer to the front wall is substantially the same as the distance between the back wall and the bar of the first portion closer to the back wall. The two bars are positioned at substantially the same vertical position, i.e. if there is a difference in the vertical positions of the two bars, the difference is small enough to allow stable mounting of the instruments. The two bars positioned at the same vertical positions give the best stability.

US5571488 discloses an apparatus for cleaning dental instruments having internal channels. The apparatus includes a closable chamber for receiving the instruments and a cassette for receiving the instruments. The cassette is placeable in the closable chamber. The cassette has a backwall containing bores. The chamber has nozzle arms which pass through the bores when the cassette is introduced into the chamber. During use, water is sprayed from these nozzle arms. Instruments whose inside comprise media channels are attached to an adapter of the cassette. Instruments having no media channels are placed on the floor or base of the cassette. The cassette is provided with one or more outlet openings at the floor, for the discharge of the supplied cleaning fluids. The apparatus of US5571488 does not comprise an instrument mounting member extending between two side walls. With the apparatus of US5571488, instruments whose inside comprise media channels must be attached to the adapter, which adapter does not extend between two side walls. The adapter does not comprise a portion formed of two bars positioned at substantially the same vertical position with a space therebetween, which portion is above an opening for spraying of cleaning liquid.

WO2010/128408 discloses a mobile apparatus for sterilizing a plurality of surgical trays within an autoclave. The surgical trays are placed on a shelf in the apparatus to be sterilized. EP1839683 discloses a tray for surgical instruments having a lumen. The instruments are washed by a duct connected to a water supply.

US4577755 discloses a surgical instrument tray that has a base with a raised portion to support lever members of ring-handled surgical instruments. The base is formed to have an end portion angulating away from the base with slots formed therein sized to receive the ring handles attached to the lever members of the surgical instruments to hold the instruments in an upstanding manner. The base may be provided with holes to permit sterilizing material to flow therethrough. Apertures are positioned in opposite side members proximate the raised portion through which retaining members are positioned to hold the surgical instruments in the tray and to hold the working member portion of the surgical instrument separate to facilitate sterilization. The retaining members are arcuate in shape in order to apply a spring-like force to retain selectively surgical instruments in the tray and to selectively hold the lever member of the instrument apart so that the working members of the surgical instrument are separate.

None of these documents discloses a container having the combination of technical features according to the present invention.

The width of the space between the two bars should be chosen to allow spraying of the cleaning liquid to the pivot points. For example, the distance between the two bars may preferably range between 1-4 cm, more preferably, 1.2-3 cm, even more preferably 1.5-2.5 cm. The thickness of the bars should be chosen in combination with the distance between the two bars to allow stable mounting of the medical or dental instruments in an open state. For example, the thickness of the bars may be 0.1-1 cm, preferably 0.2-0.5 cm. The distance between the two bars and the thickness of the bars may be constant along the first portion. Alternatively, the distance between the two bars and/or the thickness of the bars may vary along the first portion. This allows mounting instruments of different sizes at their respective suitable positions on the first portion of the instrument mounting member.

The instrument mounting member may be attached to the side walls by any known means. In the cases where the instrument mounting member is fixed to the side walls, the instrument mounting member may e.g. be welded to the side walls. Preferably, however, the instrument mounting member is removably attached to the container. In this case, for example, the side walls may be provided with slots in which the edge of the removable instrument mounting member may be inserted. The walls of the container are in this case flexible enough to allow inserting and removing the instrument mounting member. Many suitable arrangements for removable attachment are known to the skilled person and are not described here in detail.

The instrument mounting member should preferably be positioned at a vertical position, i.e. a height, which ensures that the mounted instruments do not protrude from the container from the opening at the bottom below the first portion. For example, the instrument mounting member may be positioned at a height of 5-15 cm, preferably 6-12 cm or 7-10 cm.

For ease of mounting and removing of the instruments from the mounting member, one or both of the back wall and the front wall preferably has a height smaller than the height of the side walls. Preferably, one or both of the back wall and the front wall has a height smaller than the vertical position of the mounting member. For example, the height of the side walls may be 10-20 cm. For example, the height of the back wall and the front wall may be 10-70% of the height of the side walls, more preferably 15-60%, more preferably 20-40%, of the height of the side walls. The height of the back wall and the front wall may e.g. be 2-10 cm, preferably 3-6 cm.

When the height of the wall varies depending on the position, the largest height of the wall is understood as the height of the wall.

The first portion of the instrument mounting member is preferably provided with one or more dividers. This provides each instrument a discreet position on the first portion. The dividers may take form of e.g. protrusions on the bars.

The first portion of the instrument member may have a length of e.g. 5-30 cm, more preferably 7-20 cm, more preferably 8-15 cm.

The container according to the present invention is particularly advantageous for use in a washing apparatus which is arranged to upwardly spray cleaning liquids. Some washing apparatuses of this type are arranged to additionally spray cleaning liquids downwardly for more powerful washing. For suitable use with these apparatuses, the container according to the present invention may be arranged to further comprise a part suitable for washing with a cleaning liquid sprayed from above. Accordingly, in some embodiments, the instrument mounting member may further comprise a second portion defining at least one instrument inserting opening and the container comprises a bottom wall below the instrument inserting area. It will however be appreciated that the bottom wall does not cover the entire bottom of the container, since the container requires an opening for spraying from below.

The instruments inserted in the instrument inserting opening are washed with cleaning liquid sprayed from above. The second portion is used for instruments which do not have two branches pivotable with respect to each other, i.e. instruments which cannot be mounted on the first portion. These instruments may include instruments such as mirrors, picks, scrapers, files, scalpels, and other small hand tools. The second portion defines and encloses an area in which these types of instruments can be inserted. These types of instruments are supported by the bottom wall below the instrument inserting opening and lean against the second portion defining the instrument inserting opening. It will be appreciated that the instrument inserting opening is an opening which has a size suitable for easily inserting and removing dental and medical instruments. For example, the instrument inserting opening has an area of 3-10 cm². The second portion may have a length along the back side of e.g. 1-3 cm.

The bottom wall may be removable. This allows using different types of instrument mounting members with one container depending on the desired situation. An instrument mounting member without the second portion may preferably be used with the container without the bottom wall and an instrument mounting member with the second portion may preferably be used with the same container but with the bottom attached.

The bottom wall may be provided with an upwardly extending edge to ensure that the instruments inserted in the instrument inserting opening do not fall off from the container.

The container may be further provided with additional means to ensure that the instruments inserted in the instrument inserting opening do not fall off from the container. For example, a further member may be provided below or above the second portion which defines an opening similar to the instrument inserting opening of the second portion. The instruments can then be inserted in two openings.

The instrument inserting opening may have any shape, such as circular, rectangular, square, semi-circular, quarter circular etc.

The side walls, the back wall and/or the front wall of the container may be perforated. The perforation reduces the weight of the container, which allows easier handling of the container.

Preferably, each of the side walls is provided with a handle. The handle preferably protrudes outwardly and extends along the top rim of the side wall. This allows easy carrying of the container.

Preferably, the handles are arranged such that the handles do not overlap with each other when viewed in the direction along the longitudinal direction of the instrument mounting member. This allows the lower side of the handles to be washed by the cleaning liquids sprayed from below when the containers are placed side by side with a space for one handle in between. Side by side is herein meant that the outer surfaces of the side walls of neighboring containers face each other. In one example, the handle on one side wall is provided close to the back wall and the handle on the other side wall is provided close to the front wall. The handles have a length less than half of the length of the top rim of the side wall. If it is desired to place as many containers as possible in a rack, the containers may be placed with little space in between such that the handle of a container extend above the neighboring container.

The container may be made of any material suitable for use in a medical instrument washer. Suitable materials include stainless steel.

The container containing the instruments may be placed in the working space of the dentists and doctors and their assistants. After the instruments are used, the instruments are placed in the container and the container is brought to the washing apparatus. The container can be carried without touching the used instruments which are potentially harmful. After the instruments are washed in the container, the container may be brought to the working space without the instruments being touched. This ensures that the instruments are maintained clean until they are used. The clean instruments can be taken directly from the container to be used on the patients.

Another aspect of the invention provides a rack containing a plurality of containers according to the present invention arranged side by side. Preferably, each of the side walls is provided with a handle protruding outwardly and extending along the top rim of the side wall and the handles are provided such that the handles do not overlap with each other when viewed in the direction along the instrument mounting member.

Another aspect of the invention provides a method of washing dental instruments or medical instruments in a washing machine provided with a spray means for spraying cleaning liquid upward, comprising the steps of: a) placing the instruments on the instrument mounting member of the container according to the present invention, b) placing the container in the washing machine above the spray means, c) spraying the cleaning liquid at the instruments.

Step b) may comprise the step of placing a plurality of the containers side by side in a rack and placing the rack in the washing machine above the spray means.

Another aspect of the invention provides use of the container according to the present invention in a washing machine provided with a spray means for upwardly spraying cleaning liquid, for washing dental instruments or medical instruments.

It is noted that the invention relates to all possible combination of features recited in the claims or the description.

These and other aspects of the invention will be apparent from and elucidated with reference to the drawings in which:
Figure 1 schematically illustrates a perspective view of an embodiment of the container according to the present invention;
Figure 2 schematically illustrates a perspective view of a further embodiment of the container according to the present invention;
Figure 3 schematically illustrates a cross section of the container according to the present invention in which a dental plier is received;
Figure 4(a) schematically illustrates an embodiment of the instrument mounting member according to the present invention;
Figure 4(b) schematically illustrates a further embodiment of the instrument mounting member according to the present invention;
Figure 5 schematically illustrates a front view of an embodiment of the container according to the present invention;
Figure 6 schematically illustrates a top plan view of an embodiment of the rack containing a plurality of containers according to the present invention
Figure 7 illustrates a perspective view of a further embodiment of the container according to the present invention and
Figure 8 is a scheme illustrating a typical method in which the container according to the invention is used.

It is noted that the figures are only schematic and are not drawn to scale. Same reference numbers have been used for same elements in different embodiments where possible.

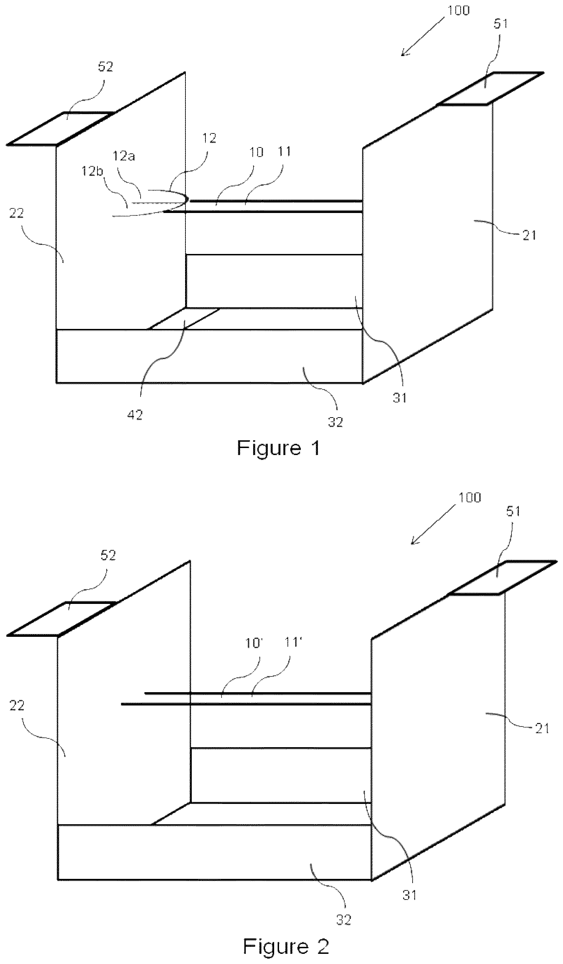

Referring to Figure 1, a perspective view of an embodiment of the container 100 according to the present invention is shown. The container 100 comprises a first side wall 21 and a second side wall 22 connected by a back wall 31 and a front wall 32. A first handle 51 is provided at the top of the first side wall 21 and a second handle 52 is provided at the top of the second side wall 22. The first handle 51 has a length less than half of the first side wall 21 and is provided at the front side. The second handle 52 has a length less than half of the second side wall 22 and is provided at the back side.

The container 100 further comprises an instrument mounting member 10 extending between the first and the second side walls 21 and 22. The instrument mounting member 10 comprises a first portion 11 formed of two parallel bars. The two parallel bars are attached to the first side wall 21. The instrument mounting member 10 further comprises a second portion 12 attached to the second side wall 22. The second portion 12 defines two instrument inserting openings 12a and 12b.

The container 100 comprises a bottom wall 42 extending from the bottom of the second side wall 22 towards the bottom of the first side wall 21 and connected to the bottom of the front wall 31 and the back wall 32. The bottom wall 42 extends only partly so that it is present below the instrument inserting openings 12a and 12b but there is an opening below the first portion 11.

Figure 2 shows a perspective view of a further embodiment of the container according to the present invention. This embodiment is substantially the same with the embodiment of figure 1 except that the instrument mounting member 10 of Figure 1 has been replaced by another instrument mounting member 10' and the container 100 does not comprise a bottom wall. The instrument mounting member 10' consists only of the first portion 11.

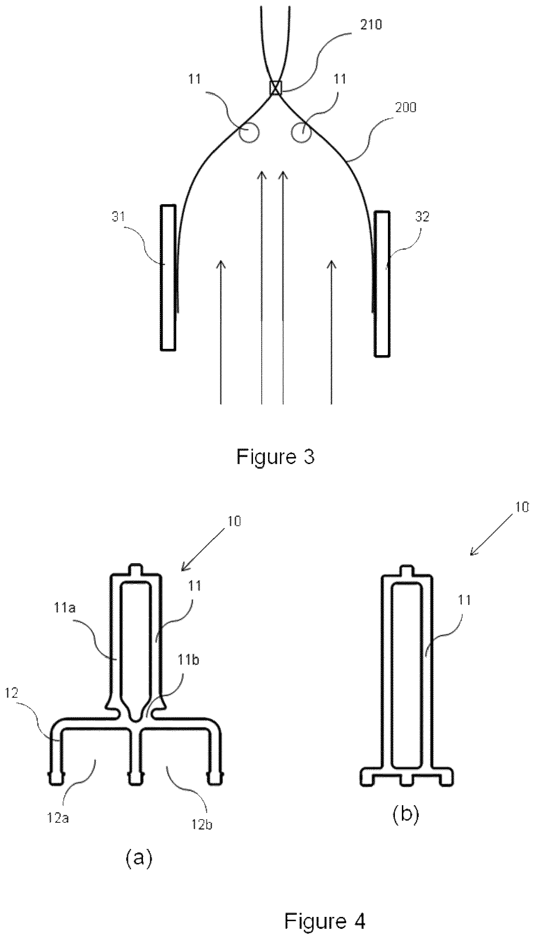

Referring to Figure 3, a cross section of the container is shown in which a dental instrument 200 (a pair of pliers) is mounted on the bars 11 of the instrument mounting member. The plier is in an open position and the handles of the plier rest on the bars 11. During the washing procedure, the pivot point 210 is directly sprayed from below with cleaning liquids as indicated by arrows.

Referring to Figure 4(a), a top view of an embodiment of the instrument mounting member 10 according to the present invention is shown. The instrument mounting member 10 comprises a first portion 11 comprising two bars closed at its both ends. At one end the first portion 11 is provided with a protrusion which is to be inserted into a slot on the side wall of the container. The first portion 11 comprises two bars which form one part 11a with a larger space in between and another part 11b with a smaller space in between. The part 11a of the first portion 11 may be used for mounting larger instruments and the part 11b of the first portion 11 may be used for mounting smaller instruments. The instrument mounting member 10 further comprises a second portion 12 which define two instrument inserting openings 12a and 12b. The edges of the second portion 12 are to be inserted into slots on the side wall of the container.

Figure 4(b) shows a further embodiment of the instrument mounting member 10 according to the present invention. The instrument mounting member 10 comprises a first portion 11 closed at its both ends and no second portion defining instrument inserting openings. The protrusions provided at the ends of the first portion 11 are to be inserted into slots on the side walls of the container.

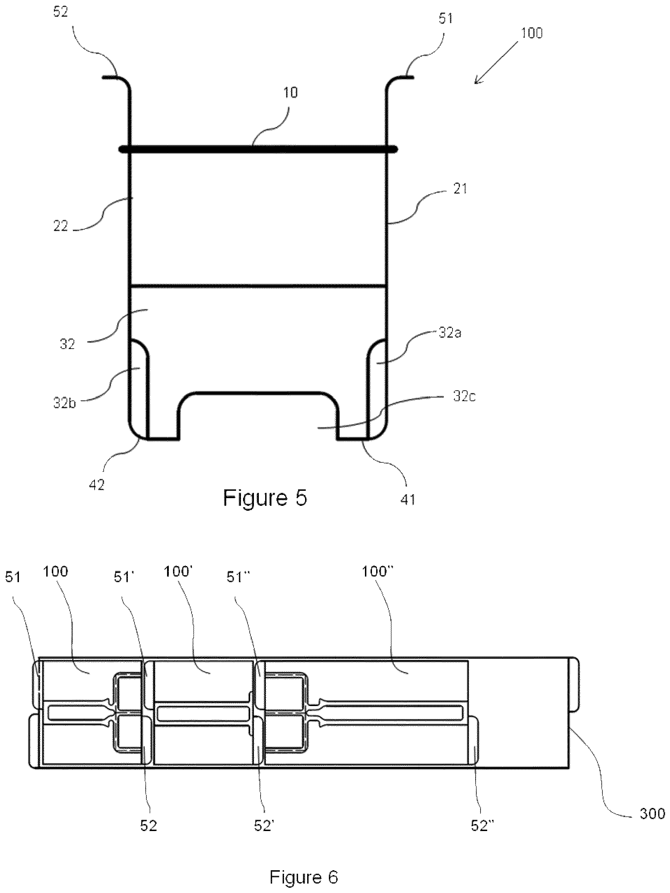

Figure 5 shows a front view of an embodiment of the container 100 according to the present invention. The container 100 comprises two bottom walls 41 and 42, each connected to the first side wall 21 and the second wall, respectively. The front wall 32 has three openings 32a, 32b and 32c. These openings 32a-32c make the container more lightweight.

Figure 6 shows a top plan view of an embodiment of a rack containing a plurality of containers according to the present invention. The rack 300 contains three different embodiments of the container 100, 100' and 100" according to the present invention arranged side by side. The containers 100, 100' and 100" each comprises different types of the instrument mounting member. The containers 100 and 100" have a first portion as well as a second portion defining instrument inserting openings. The bottom walls below the instrument inserting openings are shown by dotted line.

The container 100 comprises handles 51 and 51' similarly arranged as the embodiment of figure 1, i.e. the handles are arranged in such a way that the handles do not overlap with each other when viewed in the direction along the longitudinal direction of the instrument mounting member. The two further containers 100' and 100" also have handles arranged similarly to the container 100. Due to the arrangement of the handles, the containers 100 and 100' can be arranged side by side so that the handle 52 of the container 100 does not overlap with the handle 51' of the container 100'. This allows the lower side of the handles 51' and 52 to be washed by the cleaning liquids sprayed from below. The same applies to the relationship between the containers 100' and 100".

The embodiment in figure 6 illustrates a situation in which the handles can be washed well, but if it is desired to place as many containers as possible in a rack, the containers may be placed with little space in between.

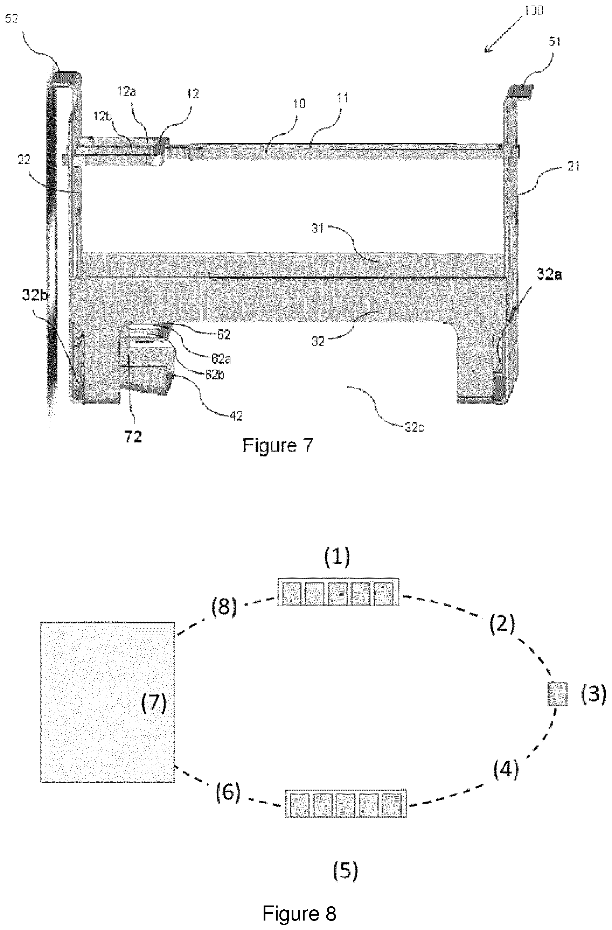

Figure 7 illustrates a perspective view of a further embodiment of the container according to the present invention. This embodiment is substantially the same with the embodiment of figure 1 except for the following.

In the embodiment of Figure 7, The bottom wall 42 is provided by a support means 72 provided below the second portion 12. The support means 72 is attached to the side wall 22. The support means 72 comprises the bottom wall and a portion 62 which defines openings 62a, 62b similar to the instrument inserting openings 12a,12b of the second portion 12. Instruments may be inserted in the instrument inserting openings 12a, 12b and openings 62a,62b and be supported by the bottom wall 42. The bottom wall 42 is provided with upwardly extending edges to ensure that the instruments do not fall off from the container 100.

The front wall 32 in the embodiment of Figure 7 has three openings 32a, 32b and 32c. The back wall 31 may also have similar openings. The side walls 21,22 also have openings. These openings are advantageous for making the container lightweight.

Figure 8 is a scheme illustrating a typical method in which the container according to the invention is used by an orthodontist or a doctor. The method includes eight phases in which:
(1) A rack comprising containers containing dental or medical instruments is stored in a clean area, waiting to be used. The rack, the containers and the instruments are all clean.
(2) A container with the clean instruments is picked up from the rack in the clean area and moved to an area to be used with the patient.
(3) The instruments are used on the patient. After use, the contaminated instruments are placed back in the container.
(4) The container with the contaminated instruments is moved to a place for the contaminated instruments.
(5) The container is placed in a rack for the contaminated instruments, together with other containers with contaminated instruments.
(6) The rack comprising the containers with contaminated instruments is moved into a washing machine.
(7) The washing machine cleans and disinfects the rack, the containers and the contaminated instruments.
(8) The washing apparatus is off-loaded and the rack comprising the containers containing the instruments is moved to the clean area.
(9) = (1)

This method increases safety for personnel by eliminating the need to take out instruments for cleaning; the instruments are only taken out of the container when used with the patient; see (3). Instruments such as pliers are stored open in the container allowing these instruments to be oiled without taken them out. As the instruments are no longer touched by personnel, the risk for contamination of the instruments by the personnel is also eliminated - safety for the patient therefore increases too.

This method also increases efficiency. After the washing is completed, there is no need for the personnel to touch the instruments to move them to a clean place for storage. When the instruments are to be used, instruments do not have to be collected and put on e.g. a tray to be used with the patient. Preparation for use of the instruments can be done only by bringing the container to the place where the instruments are needed. After use, instruments do not have to be put in cassettes before putting them in the washing apparatus. The time saved by this procedure has a significant impact on availability of professional staff (the overall efficiency of the process is increased with 15-20%) and therefore allows more patients to be helped in the same amount of time. The quality of work for the staff is hence also improved.

## Claims

1. A container (100) for dental instruments and medical instruments, said instruments having two branches that are pivotably arranged with respect to each other to allow said instruments to assume an open state and a closed state, wherein the container comprises a bottom, two side walls (21,22) facing each other, a back wall (31) and a front wall (32) connected to the two side walls and facing each other, and an instrument mounting member (10) extending between the two side walls and attached to the side walls, wherein the instrument mounting member comprises a first portion (11) being formed of two bars positioned at the same height with respect to the bottom with a space therebetween, the two bars being configured to enable mounting of said instruments in an open state on the first portion of the instrument mounting member, **characterized in that** the back wall and the front wall are arranged at a distance with respect to each other that is in accordance with a space between the two branches of said instruments when in an open state and configured to limit rotational movement of said instruments when mounted in an open state on the first portion of the instrument mounting member, the bottom of the container being provided with an opening and the first portion of the instrument mounting member being arranged above the opening,
wherein the opening is configured to allow, in use of the container, cleaning liquid to be sprayed unimpeded and directly onto the instruments that are mounted in an open state on the first portion of the instrument mounting member.

2. The container according to claim 1, wherein instrument mounting member is removably attached to the side walls.

3. The container according to claim 1 or 2, wherein the distance between the back wall and the front wall is 7-12 cm.

4. The container according to any one of the preceding claims, wherein the distance between the two bars ranges between 1-4 cm.

5. The container according to any one of the preceding claims, wherein the instrument mounting member is positioned at a height of 5-15 cm.

6. The container according to any one of the preceding claims, wherein the instrument mounting member is provided with one or more dividers.

7. The container according to any one of the preceding claims, wherein the container further comprises a second portion (12) defining at least one instrument inserting opening (12a, 12b) and the container further comprises a bottom wall (42) below the instrument inserting opening.

8. The container according to any one of the preceding claims, wherein the instrument inserting opening has an area of 3-10 cm².

9. The container according to claims 7 or 8, wherein the bottom wall is removable.

10. The container according to any one of the preceding claims, wherein each of the side walls is provided with a handle (51, 52) protruding outwardly and extending along the top rim of the side wall.

11. The container according to claim 10, wherein the handles are arranged such that the handles do not overlap with each other when viewed in the direction along the longitudinal direction of the instrument mounting member.

12. A rack containing a plurality of containers according to claim 11 arranged side by side.

13. A method of washing dental instruments or medical instruments in a washing machine provided with a spray means for spraying cleaning liquid upward, comprising the steps of:
a) placing the instruments on the instrument mounting member of the container according to any one of claims 1-11,
b) subsequently placing the container in the washing machine above the spray means and
c) spraying the cleaning liquid at the instruments.

14. The method according to claim 13, wherein step b) comprises the step of placing a plurality of the containers side by side in a rack and placing the rack in the washing machine above the spray means.

15. Use of the container according to any one of claims 1-11 in a washing machine provided with a spray means for upwardly spraying cleaning liquid, for washing dental instruments or medical instruments.

## Patentansprüche

1. Behälter (100) für zahnärztliche Instrumente und medizinische Instrumente, wobei die Instrumente zwei Schenkel aufweisen, die schwenkbar in Bezug aufeinander angeordnet sind, um zu ermöglichen, dass die Instrumente einen offenen Zustand und einen geschlossenen Zustand annehmen, wobei der Behälter einen Boden, zwei Seitenwände (21, 22), die einander gegenüberliegen, eine Rückwand (31) und eine Vorderwand (32), die mit den zwei Seitenwänden verbunden sind und einander gegenüberliegen, und ein Instrumentenanbringungselement (10), das sich zwischen den zwei Seitenwänden erstreckt und an den Seitenwänden befestigt ist, umfasst, wobei das Instrumentenanbringungselement einen ersten Abschnitt (11) umfasst, der von zwei Stäben gebildet wird, die bei der gleichen Höhe in Bezug auf den Boden mit einem Abstand zwischen denselben angeordnet sind, wobei die zwei Stäbe dafür konfiguriert sind, ein Anbringen der Instrumente in einem offenen Zustand am ersten Abschnitt des Instrumentenanbringungselements zu ermöglichen, **dadurch gekennzeichnet, dass** die Rückwand und die Vorderwand in einer Entfernung in Bezug aufeinander, die sich in Übereinstimmung befindet mit einem Abstand zwischen den zwei Schenkeln der Instrumente, wenn sie sich in einem offenen Zustand befinden, angeordnet und dafür konfiguriert sind, eine Drehbewegung der Instrumente zu begrenzen, wenn sie in einem offenen Zustand am ersten Abschnitt des Instrumentenanbringungselements angebracht sind, wobei der Boden des Behälters mit einer Öffnung versehen ist und der erste Abschnitt des Instrumentenanbringungselements oberhalb der Öffnung angeordnet ist,
wobei die Öffnung dafür konfiguriert ist, bei Verwendung des Behälters zu ermöglichen, dass Reinigungsflüssigkeit ungehindert und unmittelbar auf die Instrumente gesprüht wird, die in einem offenen Zustand am ersten Abschnitt des Instrumentenanbringungselements angebracht sind.

2. Behälter nach Anspruch 1, wobei das Instrumentenanbringungselement entfernbar an den Seitenwänden befestigt ist.

3. Behälter nach Anspruch 1 oder 2, wobei die Entfernung zwischen der Rückwand und der Vorderwand 7 bis 12 cm beträgt.

4. Behälter nach einem der vorhergehenden Ansprüche, wobei die Entfernung zwischen den zwei Stäben von 1 bis 4 cm reicht.

5. Behälter nach einem der vorhergehenden Ansprüche, wobei das Instrumentenanbringungselement bei einer Höhe von 5 bis 15 cm angeordnet ist.

6. Behälter nach einem der vorhergehenden Ansprüche, wobei das Instrumentenanbringungselement mit einer oder mehreren Teilungsvorrichtungen versehen ist.

7. Behälter nach einem der vorhergehenden Ansprüche, wobei der Behälter ferner einen zweiten Abschnitt (12) umfasst, der wenigstens eine Instrumenteneinsetzöffnung (12a, 12b) definiert, und der Behälter ferner eine Bodenwand (42) unterhalb der Instrumenteneinsetzöffnung umfasst.

8. Behälter nach einem der vorhergehenden Ansprüche, wobei die Instrumenteneinsetzöffnung eine Fläche von 3 bis 10 cm² aufweist.

9. Behälter nach Anspruch 7 oder 8, wobei die Bodenwand entfernbar ist.

10. Behälter nach einem der vorhergehenden Ansprüche, wobei jede der Seitenwände mit einem Handgriff (51, 52) versehen ist, der nach außen vorspringt und sich entlang des oberen Randes der Seitenwand erstreckt.

11. Behälter nach Anspruch 10, wobei die Handgriffe derart angeordnet sind, dass die Handgriffe nicht miteinander überlappen, wenn sie in der Richtung entlang der Längsrichtung des Instrumentenanbringungselements betrachtet werden.

12. Gestell, das eine Vielzahl von Behältern nach Anspruch 11, die nebeneinander angeordnet sind, enthält.

13. Verfahren zum Waschen von zahnärztlichen Instrumenten und medizinischen Instrumenten in einer Waschmaschine, die mit einem Sprühmittel zum Sprühen von Reinigungsflüssigkeit nach oben versehen ist, wobei das Verfahren die folgenden Schritte umfasst:
a) Anordnen der Instrumente auf dem Instrumentenanbringungselement des Behälters nach einem der Ansprüche 1 bis 11,
b) anschließendes Anordnen des Behälters in der Waschmaschine oberhalb des Sprühmittels und
c) Sprühen der Reinigungsflüssigkeit auf die Instrumente.

14. Verfahren nach Anspruch 13, wobei Schritt b) den Schritt des Anordnens einer Vielzahl von Behältern nebeneinander in einem Gestell und des Anordnens des Gestells in der Waschmaschine oberhalb des Sprühmittels umfasst.

15. Verwendung des Behälters nach einem der Ansprüche 1 bis 11 in einer Waschmaschine, die mit einem Sprühmittel zum Sprühen von Reinigungsflüssigkeit nach oben versehen ist, zum Waschen von zahnärztlichen Instrumenten und medizinischen Instrumenten.

## Revendications

1. Récipient (100) pour instruments dentaires et instruments médicaux, lesdits instruments ayant deux branches qui sont agencées de façon pivotante l'une par rapport à l'autre pour permettre auxdits instruments d'adopter un état ouvert et un état fermé, dans lequel le récipient comprend une partie inférieure, deux parois latérales (21, 22) se faisant face, une paroi arrière (31) et une paroi avant (32) raccordées aux deux parois latérales et se faisant face, et un élément de montage d'instruments (10) s'étendant entre les deux parois latérales et fixé aux parois latérales, dans lequel l'élément de montage d'instruments comprend une première partie (11) étant formée de deux barres positionnées à la même hauteur relativement à la partie inférieure avec un espace entre celles-ci, les deux barres étant configurées pour permettre le montage desdits instruments dans un état ouvert sur la première partie de l'élément de montage d'instruments, **caractérisé en ce que** la paroi arrière et la paroi avant sont agencées à une distance l'une par rapport à l'autre qui est conforme à un espace entre les deux branches desdits instruments dans un état ouvert et configuré pour limiter le mouvement rotatif desdits instruments lorsqu'ils sont montés dans un état ouvert sur la première partie de l'élément de montage d'instruments, la partie inférieure du récipient étant dotée d'une ouverture et la première partie de l'élément de montage d'instruments étant agencée au-dessus de l'ouverture,
dans lequel l'ouverture est configurée pour permettre, durant l'utilisation du récipient, du liquide de nettoyage à pulvériser sans entrave et directement sur les instruments qui sont montés dans un état ouvert sur la première partie de l'élément de montage d'instruments.

2. Récipient selon la revendication 1, dans lequel l'élément de montage d'instruments est fixé de façon amovible aux parois latérales.

3. Récipient selon la revendication 1 ou 2, dans lequel la distance entre la paroi arrière et la paroi avant est de 7 à 12 cm.

4. Récipient selon l'une quelconque des revendications précédentes, dans lequel la distance entre les deux barres se trouve dans la plage de 1 à 4 cm.

5. Récipient selon l'une quelconque des revendications précédentes, dans lequel l'élément de montage d'instruments est positionné à une hauteur de 5 à 15 cm.

6. Récipient selon l'une quelconque des revendications précédentes, dans lequel l'élément de montage d'instruments est doté d'un ou plusieurs séparateurs.

7. Récipient selon l'une quelconque des revendications précédentes, dans lequel le récipient comprend en outre une seconde partie (12) définissant au moins une ouverture d'insertion d'instruments (12a, 12b) et le récipient comprend en outre une paroi inférieure (42) au-dessous de l'ouverture d'insertion d'instruments.

8. Récipient selon l'une quelconque des revendications précédentes, dans lequel l'ouverture d'insertion d'instruments a une surface de 3 à 10 cm².

9. Récipient selon la revendication 7 ou 8, dans lequel la paroi inférieure est amovible.

10. Récipient selon l'une quelconque des revendications précédentes, dans lequel chacune des parois latérales est dotée d'une poignée (51, 52) faisant saillie vers l'extérieur et s'étendant le long du bord supérieur de la paroi latérale.

11. Récipient selon la revendication 10, dans lequel les poignées sont agencées de telle sorte que les poignées ne se chevauchent pas les unes les autres lorsqu'elles sont vues dans la direction le long de la direction longitudinale de l'élément de montage d'instruments.

12. Support contenant une pluralité de récipients selon la revendication 11 agencés côte à côte.

13. Procédé de lavage d'instruments dentaires ou d'instruments médicaux dans une laveuse mécanique dotée d'un moyen de pulvérisation pour pulvériser le liquide de nettoyage vers le haut, comprenant les étapes suivantes :
a) placement des instruments sur l'élément de montage d'instruments du récipient selon l'une quelconque des revendications 1 à 11,
b) ensuite placement du récipient dans la laveuse mécanique au-dessus du moyen de pulvérisation et
c) pulvérisation du liquide de nettoyage sur les instruments.

14. Procédé selon la revendication 13, dans lequel l'étape b) comprend l'étape de placement d'une pluralité de récipients côte à côte dans un support et de placement du support dans la laveuse mécanique au-dessus du moyen de pulvérisation.

15. Utilisation du récipient selon l'une quelconque des revendications 1 à 11 dans une laveuse mécanique dotée d'un moyen de pulvérisation pour pulvériser vers le haut le liquide de nettoyage, pour laver les instruments dentaires ou les instruments médicaux.
